# EUROPEAN PATENT APPLICATION

(11) **EP 4 574 204 A2**
(43) Date of publication of application: **25.06.2025**
(21) Application number: 25173852.2
(22) Date of filing: 28.11.2022
(51) Int. Cl.: A61N 5/06

(54) **ELECTRO-OPTICAL IRRADIATION DEVICE**

(30) Priority: 26.11.2021 EP 21210657
(62) Divisional of application: 22822924.1
(71) Applicant: Sunled Life Science B.V., 1098XG Amsterdam (NL)
(72) Inventor: BERENDS, Anne Claire, 3551 TJ UTRECHT (NL); KRAMES, Michael, Kentfield, CA 94904 (US)
(74) Representative: Hoyng Rokh Monegier B.V.

(57) **Abstract**

An apparatus for a display system, capable of providing a photobiomodulation effect in a user of the system. The apparatus has at least one electro-optical element (EL1) arranged to emit electromagnetic radiation having a peak emission wavelength between 700-1400 nm, a control circuit (120, 130) configured to adjust emission characteristics of the electro-optical element, and a driver circuit (110) configured to receive an input from the control circuit (120, 130) and to drive the electro optical element with a pulsed driving current based on the input. The apparatus is configured such that the at least one electro optical element (EL1) emits said electromagnetic radiation only if a visual stimulus at a luminance above 10 cd/m² is present.

## Description

### FIELD OF THE INVENTION

The invention relates to the field of electro-optical irradiation devices having at least one electro-optical element arranged to emit electromagnetic radiation having a peak emission wavelength between 700-1000 nm. In practice, application areas of such devices can be manifold, since they may be incorporated in luminaires, screen devices, mobile devices, mirrors, etc, which may be used in daily context for a user. For some devices, there is an aim to provide enhanced lighting functionality, which may also have a beneficial effect on the user, for instance, by applying the indicated emission wavelength to obtain a photo biomodulation effect, which is a known light therapy that is described in the literature as stimulating, healing or regenerating for human users.

### BACKGROUND OF THE INVENTION

PBM devices in prior art utilize equipment that administer PBM radiation either by high powers, or by high intensities employing near-field excitation of the skin or treatment area (proximity devices). These specifications render the PBM effect exclusively in specialized devices, often focused on medical or therapeutic purposes. Similarly, many systems are designed to irradiate the entire body and require dedicated delivery which requires the user to remain immobilized for certain periods of time in order to receive a PBM response. These limitations prevent the general public's access to PBM health benefits.

In the prior art it is also known to provide such devices, e.g. as described in PCT Application WO 2020/119965. Exposure of certain amounts of red (R) to near-infrared (NIR) radiation to living organisms has been shown to induce biological and/or biochemical responses which lead to beneficial effects such as stimulating healing, relieving pain, and reducing inflammation.

To employ this photobiomodulation (PBM) technique, a conventional approach has been to deliver the required radiation through a dedicated device applied directly or very near the surface of the skin.

The concept of delivering PBM effects through free-space radiation within the context of a general purpose illumination system was earlier proposed by the Applicant. To deliver the required irradiated intensity levels, high peak pulsing of the R/NIR radiation sources is typically employed, e.g. as described for example in PCT Application WO2020/119965. Various ranges of pulse-widths and frequencies are described to deliver a targeted PBM dose expected to provide beneficial effects. Various techniques for driving an electro-optical element with a pulse width, frequency and radiation level, in order to obtain a targeted PBM dose are disclosed. One or more of these elements may also be applied in the present invention. For example, WO2020/119965 discloses a lighting arrangement. The lighting arrangement may comprise a radiation source, a light source and a driver circuit. The light source may be adapted to emit visible light. In an example, the light source may be capable of or suitable for emitting visible light having a color point in a CIE chromaticity space, which color point has a distance less than 10 Standard Deviations of Color Matching (SDCMs) to a black body line in said color space. The driver circuit may be adapted to provide a driving current, which may be pulsed and may have a duty cycle of not greater than 20%. The lighting arrangement may be adapted to provide the driving current to the radiation source but not the light source.

One reason to provide a PWM modulated signal is to achieve at the one hand substantially high irradiation levels, but on the other hand reducing the average irradiation, to prevent overheating and other potential harmful effects.

Another reason of the PWM modulation is to efficiently generate the required irradiation levels, which are also typically generated with limited energy supply means, especially in handheld, battery operated devices. By suitable choices of radiation levels and modulation frequencies, a balanced use of the available electrical energy can be obtained without draining the battery too soon. Pulsing for PBM is effective because of the threshold effect regarding inducing it. For example, it is generally accepted that an irradiation intensity of > 0.1 mW/cm², preferably > 0.4 mW/cm², and more preferably > 1 mW/cm² at the skin's surface is required to induce a meaningful PBM effect. As a comparison, continuous illumination to achieve 500 lux (a typical target for office lighting) using white light results in less than 0.2 mW/cm² irradiation intensity. Due to the higher intensity requirement for PBM, it can be prohibitive from a cost and energy use point of view to continually deliver radiation meaningful for a PBM effect.

Instead, it can be advantageous to pulse a R and/or NIR emitter to overcome the irradiation intensity threshold described above and deliver a meaningful PBM dose, while maintaining an average power dissipation that avoids overheating the emitter in the case that sustained high power density operation of the emitting device would cause thermal management challenges and possibly radiation output degradation and/or reduced operating lifetime.

A further option to reduce the energy consumption of the enhanced lighting element is to limit the radiation to a directed beam, suitable for direct radiation in a limited etendue, so no radiation energy is wasted since it will not irradiate the user.

To this end, additionally, the PBM irradiation intensity may be increased by focusing the light in a radiation pattern narrower than that required for general illumination. That is, unlike general illumination, in which all areas of a built environment require some level of irradiation in order to be seen, PBM radiation is only functional when directed (possibly, with use of reflectors or refractors) to the subject target or portion of the subject target, which may be located in a designated location, such as in a chair at a desk, or in a bed in a hospital) room. This allows focusing of the PBM radiation, to create a higher irradiated intensity while using less total radiation, according to the inverse-square law of electromagnetic radiation.

Nevertheless, in the prior art, there is still a need for optimization of the irradiation, wherein a user may be provided with enhanced lighting effects in an integrated fashion of other uses of the device, and without being exposed to radiation levels that could still produce potential harmful effects.

WO 2021/026218 describes a PBM application in the field of treatments for central nervous system disorders. Where these disorders include an inflammatory component, light is administered at wavelengths which decrease inflammation in the brain. Where these disorders are caused by poor vascularization, light is administered at wavelengths which improve vascularization in the brain. In an example, persons are treated with NIR having a wavelength at 810 nm, energy density at 60 J/cm², and power density at 250 m W/cm².

### SUMMARY OF THE INVENTION

It is an object of the present invention to deliver a photobiomodulation (PBM) stimulus via infrared (IR) radiation to target a subject via free space. It is a further object of the invention to deliver the PBM stimulus in a manner which is cost effective and not too energy intensive. It is a further object of the invention to monitor and control a PBM dose for the target subject. Applications suitable for carrying out the invention include those where the location of a target subject is known, and/or can be monitored. This information provides an opportunity for the delivery of IR radiation at a level beyond the minimum threshold for a PBM effect. Information regarding location of the target subject also includes the length of time that a target subject stays within the area of interest, so that a dose may be calculated and monitored.

The invention is defined in the appended claims.

According to a first aspect of the disclosure, there is provided an electro-optical device capable for or suitable for providing a photobiomodulation effect in a user of the device, comprising: at least one (first) electro-optical element (EL1) arranged to emit electromagnetic radiation having a peak emission wavelength between 700-1400 nm; a control circuit configured to adjust emission characteristics of the electro-optical element; and a driver circuit configured to receive an input from the control circuit and drive the electro-optical element based on the input received from the control circuit, using a driving current that is pulsed. The control circuit may comprise a timer configured to determine an on-period for the electromagnetic radiation emitted by the electro-optical element (EL1), and/or may comprise a dose calculation unit configured to determine an accumulated dose of the electromagnetic radiation at the user. The control circuit may be configured to instruct the driver circuit to shut off the electro-optical element (EL1), if a predetermined maximum on-period is exceeded, and/or if the accumulated dose determined by the dose calculation unit indicates that a predetermined dose of the electromagnetic radiation at the user is exceeded.

The inventors have established that a PBM effect is systemic and can be achieved by irradiating a portion of a target subject's total epidermal surface area as long as it is within an acceptable irradiation intensity and dose range. The inventors have established these intensity and dose ranges, and have identified applications wherein a target subject interacts with a system for a specified duration or within a certain distance, or both, such that a PBM delivery device can be integrated within such a system and achieve a desired PBM response using low energy consumption. In some embodiments, the electro-optical device is incorporated in an object that typically has a predictable and relatively static distance with a user when it is being used. Integrating PBM inducing radiation sources into such systems in this manner provides easy access to PBM exposure to the general public through myriad systems, such as mobile phones, laptops, tablets, monitors, televisions, gaming systems, wearable devices (such as watches), (tech) gadgets (such as webcams), lighting systems, and other systems. Additionally or alternatively, in some embodiments, a sensor is arranged to detect whether the user is within a certain range and/or arranged to measure a specific distance value between the device and the user.

In this way, dose can reliably be monitored. Thus, the PBM devices may be controlled so that they are only on when they are useful, are targeted to deliver a meaningful dose PBM effect in a reasonable amount of time, and are shut off when they are no longer needed, to avoid an over-dosing situation but also to conserve energy.

According to a second aspect of the disclosure, there is provided an electro-optical device comprising: a (first) electro-optical element arranged to emit electromagnetic radiation having a peak emission wavelength between 700-1400 nm. Such an electro-optical element may be provided for enhanced irradiation functionality, in particular for providing a photobiomodulation effect in a user of the device. To this end the electro-optical element may be further provided with a driver circuit. The driver circuit may be adapted to drive the electro-optical element with a pulse-width, frequency and radiation level, e.g. using one or more elements described in WO 2020/119965, such that the electro-optical element achieves a peak irradiation intensity above 0.1 mW/cm², preferably above 1 mW/cm², but an average irradiation intensity less than 10 mW/cm² on the user. The electro-optical device may also comprise a (control) circuit configured to generate an output (and send this output to the driver circuit as its input) to make an adjustment to the electro-optical element. The input may be generated in response to an automatically generated signal related to usage. In addition, the electro-optical device may optionally comprise a second electro-optical element arranged to emit light in the visible spectrum, e.g. as described in WO 2020/119965.

The device or apparatus of the invention is capable of delivering a PBM effect to the user in simple, time efficient, energy efficient, and cost effective way. The device or apparatus is easy to use. In some embodiments, further measures are provided to avoid disturbing the user or others via human-visible flicker or image flicker. The device or apparatus of the invention is generally used in a typical geometry at a typical distance from the user for a certain minimum time duration. The typical distance and geometry allow for a narrow or directed beam of radiation towards the subject or subject area. The typical time duration allows for an even distribution of the PBM radiation over time.

Both aspects of the disclosure may comprise one or more of the following measures.

The electro-optical device may include a shut-off circuit that shuts off or tempers the first electro-optical element, in response to a signal, that may be a timer signal received after the first electro-optical element is used for a prescribed on-period. Other signals related to usage may include a registered usage parameter, such as an average expected time passed in front of the device, or average calculated dose of irradiation intensity received by the device, or a signal related to the distance between the device and the user.

In several embodiments, the electro-optical device is configured to generate an adequate but not excessive radiation intensity at the user based on the designed use of the device. In these embodiments, the beam angles of the electromagnetic radiation may further be optimized in light of such use. In this way, optimal PBM effect can be achieved with minimum energy consumption.

In an embodiment, the electro-optical device is (incorporated in) a handheld device, such as a smartphone or tablet. The driver circuit may be configured to drive the electro-optical element with a pulse-width, frequency and radiation level, such that the electro-optical element achieves a peak irradiation intensity above 0.1 mW/cm², preferably above 1 mW/cm², but an average irradiation intensity less than 10 mW/cm² on the user, e.g. at a distance of 25 cm or 30 cm from the electro-optical device. The radiation emitted by the first electro-optical element may be directed so that the radiation is emitted from the device having a radiation pattern with a full-width-at-half-power angle of less than 2x60 degrees, preferably less than 2x50 degrees, and more preferably less than 2x25 degrees.

In another embodiment, the electro-optical device is (incorporated in) a computer, such as a laptop. The driver circuit may be configured to drive the electro-optical element with a pulse-width, frequency and radiation level, such that the electro-optical element achieves a peak irradiation intensity above 0.1 mW/cm², preferably above 1 mW/cm², but an average irradiation intensity less than 10 mW/cm² on the user, e.g. at a distance of 50 cm from the electro-optical device. The radiation emitted by the first electro-optical element may be directed so that the radiation is emitted from the device having a radiation pattern with a full-width-at-half-power angle of less than 2x25 degrees, preferably less than 2x15 degrees.

In an embodiment, the second electro-optical element (EL2) is an illumination device installed for illumination or information display purposes. The driver circuit may be configured to drive the electro-optical element with a pulse-width, frequency and radiation level, such that the electro-optical element achieves a peak irradiation intensity above 0.1 mW/cm², preferably above 1 mW/cm², but an average irradiation intensity less than 10 mW/cm² on the user, e.g. at a distance of 130 cm or 170 cm from the electro-optical device. The radiation emitted by the first electro-optical element may be directed so that the radiation is emitted from the device having a radiation pattern with a full-width-at-half-power angle of less than 2x25 degrees, preferably less than 2x10 degrees. In an embodiment, the second electro-optical element (EL2) is capable of emitting white light.

In an embodiment, the first electro-optical element (EL1) is arranged to emit only when the second electro-optical element (EL2) is emitting with a luminance above 10 cd/m².

In an embodiment, the control circuit includes a proximity detection unit configured to determine a distance (d) between the user and the device, wherein the control circuit is configured to control the timer and/or the dose calculation unit in dependence on the determined distance (d).

In an embodiment, the control circuit is configured to control the driver circuit in accordance with an estimated use time per day of the electro-optical device.

In an embodiment, the control circuit is configured to control the driver circuit in dependence on a history table of device use times and/or the distance between the electro-optical device and the user.

In an embodiment, the device comprises a detection circuit configured to detect the user is in (visual) contact with the first electro-optical element, the control circuit being configured to respond to said detection and reduce a level of irradiation caused by the first electro-optical element.

In an embodiment, adjusting of the emission characteristics of the first electro-optical element by the circuit involves shutting the first electro-optical element off for a predetermined off-period.

In an embodiment, the predetermined cumulative off-period is longer than 8 hours, preferably longer than 12 hours.

In an embodiment, the peak irradiation intensity at an average distance of between 0.1 and 5 m from the (first) electro-optical element (EL1) is 0.1 mW/cm² or more, preferably between 0.4 and 50 mW/cm², and more preferably between 1 and 15 mW/cm².

In an embodiment, the irradiation intensity at an average distance of between 0.1 and 5 m from the (first) electro-optical element (EL1) is sufficient to induce a photobiomodulation effect in a human.

In an embodiment, a delivered dose over 1 hour or longer at an average distance of between 0.2 and 5 m from the (first) light source is between 0.01 and 50 J/cm², and preferably between 0.1 and 10 J/cm².

In an embodiment, the electro-optical device further comprises a light source including a second electro-optical element (EL2) and adapted to emit white light suitable for general illumination, wherein the light source is adapted to emit at least 250 lumens, preferably at least 1000 lumens, more preferably at least 2000 lumens when operating.

In an embodiment, the radiation emitted by the (first) electro-optical element (EL1) is directed by optics so that the radiation is emitted from the device having a radiation pattern with a full-width-at-half-power angle of less than 2x60 degrees, preferably less than 2x50 degrees, and more preferably less than 2x25 degrees.

In an embodiment, the radiation emitted by the (first) electro-optical element (EL1) has a radiation pattern with a full-width-at-half-power angle of 2x10 degrees or less.

In an embodiment, the electro-optical device is a display screen, comprising a first set of micro-LEDs or VCSELs arranged to emit electromagnetic radiation having a peak emission wavelength between 700-1400 nm to function as the electro-element (EL1), and a second set of micro-LEDs to function as display pixels of the screen.

In an embodiment, the pulse frequency is 100 Hz or higher.

In an embodiment, the pulse frequency is 30 Hz or a multiple of 30 Hz, preferably a common multiple of 24 Hz and 30 Hz, such as 120 Hz

In an embodiment, the electro-optical device may retrieve, store, and use information related to any of the above aspects by the use of a software application ("app") incorporated into the device.

In combination a device is provided that optimizes energy usage of the mobile device, and at the same time limits the chance of overexposure.

### BRIEF DESCRIPTION OF THE DRAWINGS

Figure 1 shows a schematic diagram of a device having first and second electro-optical elements;
Figure 2 shows a further detailed flow diagram of an adjustment circuit in response to an automatically generated signal related to usage;
Figure 3 shows a schematic diagram of relevant wavelengths emitted by said electro-optical elements;
Figures 4a-4g show various embodiments for achieving directed radiation from the first electro-optical element
Figures 5a-5c show further embodiments for achieving directed radiation;
Figures 6a-6h shows a number of driving frequencies wherein image flicker is prevented;
Figure 7 shows a schematic irradiation pattern of the first electro-optical element for a luminaire;
Figure 8a shows a schematic irradiation pattern for a handheld device;
Figure 8b shows a schematic diagram of another device having first and second electro-optical elements;
Figure 9a-9c show embodiments in which the electro-optical device is incorporated in a handheld device such as a smartphone.
Figures 10a and 10b show embodiments in which the electro-optical device is incorporated in a laptop computer.
Figure 11a shows a typical irradiation plane geometry for providing PBM radiation to a person;
Figure 11b shows an irradiance profile at the irradiation plane from figure 11a.

### DETAILED DESCRIPTION OF EMBODIMENTS

Unless otherwise defined, all terms (including technical and scientific terms) used herein have the same meaning as commonly understood by one of ordinary skill in the art to which this invention belongs as read in the context of the description and drawings. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some instances, detailed descriptions of well-known devices and methods may be omitted so as not to obscure the description of the present systems and methods. The term "and/or" includes any and all combinations of one or more of the associated listed items. It will be further understood that the terms "comprises" and/or "comprising" specify the presence of stated features but do not preclude the presence or addition of one or more other features.

Throughout the application, any means for an electro-optical assembly, which may be a light-emitting diode (LED), organic LED (OLED), laser diode (LD), vertical cavity surface emitting laser (VCSEL) or other electro-optical conversion device, may be suitable for carrying out the invention, in particular, as further clarified below.

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

The description of the exemplary embodiments is intended to be read in connection with the accompanying drawings, which are to be considered part of the entire written description. In the drawings, the size and relative sizes of systems, components, layers, and regions may be exaggerated for clarity. Embodiments are described with reference to schematic illustrations of possibly idealized and/or intermediate structures of the invention.

Photobiomodulation (PBM) involves irradiating at least a portion of a living organism at certain energy/power levels to induce biological or biochemical responses. The irradiation may be in the visible spectrum, such as red light, or in the non-visible spectrum, such as near-infrared (NIR, 700-1400 nm) or infrared (IR). There has been a significant amount of research about the medical benefits of employing PBM therapy to treat physical and psychological symptoms.

In some embodiments, use is made from a driver circuit to drive the first electro-optical element with a pulse width, frequency, and radiation level, such that the first electro-optical element achieves a peak irradiation intensity above 1 mW/cm², but an average irradiation intensity less than 10 mW/cm², exemplary driver circuit to that effect are described in previous applications:

WO2020/119965: which discloses addition of pulsed NIR or IR light to general lighting applications to induce a PBM effect while limiting the energy consumption, compared to continuous wave infra-red light.

WO2021/099642; which discloses addition of pulsed red light to general lighting applications to induce a PBM effect while limiting the energy consumption, compared to continuous wave red light.

Exemplary embodiments of such driver circuits may utilize PWM signals wherein the width of the pulses of the first pulsed current may be 0.05 ms or more. The period between pulses of the first pulsed current may be 0.05 ms or more. For example, the LEDs may be pulsed on for a period of 8 ms, and at a frequency of 10 Hz. Under the proper irradiation intensity levels, e.g., 1-10 mW/cm², a dose of 2.3-23 J/cm² is achieved.

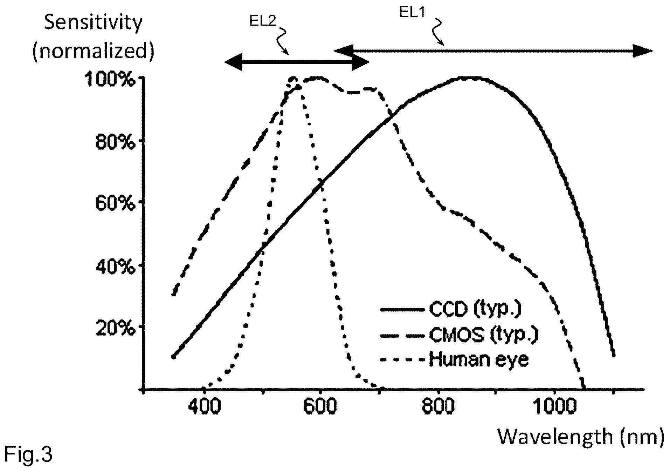

Unfortunately, while this comparative example may not exhibit human-visible flicker, it can produce image flicker that may be produced by sensors used in electronic imaging equipment including video recorders and cameras, also cameras embedded in smartphones. Such sensors can be more sensitive to infrared light than is the human eye. In particular, as shown in Fig. 3, CCD and CMOS sensors are quite sensitive to light in the wavelength regime between 600 nm and out to beyond 1000 nm. Light in this wavelength regime can interfere with the imaging function of these devices. In particular, under certain conditions, pulsing radiation in this wavelength regime can result in image flicker observable to the operator of such imaging devices.

It is desirable to choose a pulse frequency of 100 Hz or higher for pulsing PBM light sources to avoid noticeable image flicker. According to another aspect of the present disclosure, it is desirable to choose a pulse frequency for the PBM light sources which is a multiple of the typical frame rates used in imaging equipment, such as smartphone video cameras, and particularly a multiple of the frame rate of the imaging equipment to be used in the vicinity of the PBM luminaire or luminaires.

For example, if the frame rate of the relevant imaging equipment is 30 frames per second (fps), suitable choices for the pulse frequency of NIR PBM LEDs can be 30 Hz, 60 Hz, or 90 Hz, as well as any frequency at or exceeding approximately 100 Hz. If multiple imaging equipment frames rates are of concern, e.g. 24, 30 and 60 fps which may all be used in commonly available imaging equipment such as smartphones and digital cameras, then a suitable pulse frequency for pulsing PBM LEDs may be a common multiple of these frequencies, such as 120 Hz, although 100 Hz or above may also be used.

While WO2020/119965 and WO2021/099642 aim to deliver a PBM dose in an energy efficient way, the red, NIR or IR light is distributed evenly in the environment just like the visible light that is meant to illuminate the environment. The optical design for visible light radiation patterns of such devices (e.g. desk lamps or panels) differs from the ideal NIR radiation pattern for PBM: the former should illuminate the whole room or a wide area of the work place, while the latter should be directed to the subject.

A NIR beam that is focused on the subject allows for less NIR LEDs to be used, since all NIR light falls on the subject and no NIR light is used to 'illuminate' empty space. Moreover, the better the 'subject area' is defined, the better the NIR beam can be focused. Since PBM radiation is only necessary to be directed to the subject target area in order to induce a PBM effect, a more efficient delivery of PBM radiation is possible.

Electronic imaging systems such as digital cameras have sensors (e.g., CMOS or CCD sensors) that are more sensitive to deep-red and NIR radiation than the human eye. This can lead to situations in which a PBM illumination system providing pulsed radiation, while not directly visible as flickering to the human eye, will nevertheless show up as flickering when perceived through an electronic imaging system. This "image flicker" can be annoying and disruptive to important tasks, such as patient health monitoring in a hospital setting, for example. Thus, it is desired to provide a method for free-space delivery of effective PBM dosing, which does not suffer from significant levels of human-visible flicker or image flicker.

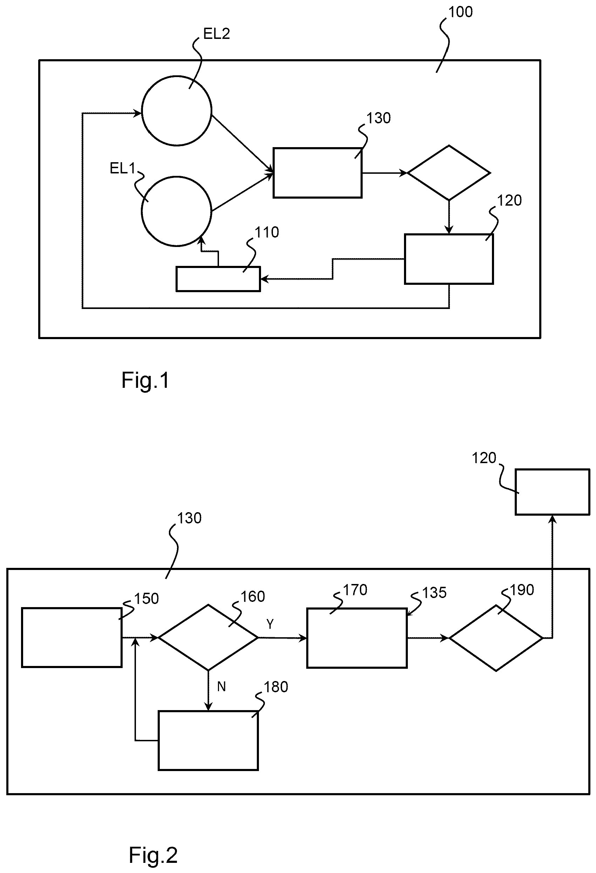

Now referring to Figure 1, a schematic diagram is shown of a device 100 having a first electro-optical element EL1 and an optional second electro-optical element EL2.

The first electro-optical element EL1 is provided arranged to emit electromagnetic radiation having a peak emission wavelength between 700-1400 nm. Such a first electro-optical element EL1 may be provided for enhanced functionality, in particular for providing a photobiomodulation effect in a user of the device 100. The first electro-optical element EL1 is arranged to emit radiation in a range that may be partially overlapping with the spectral range of the second electro-optical element EL2, but that extends to the longer (IR) wavelengths. Exemplary ranges include radiation sources having a peak irradiation intensity at an average distance of between 0.2 and 5m from the first electro-optical element EL1 of 0.1 mW/cm² or more, preferably between 0.4 and 50 mW/cm², and more preferably between 1 and 15 mW/cm². While the second radiation sources may be at any suitable distance, in particular, when combined with general illumination, and irradiation intensity at an average distance of between 0.1 and 5m from the first electro-optical element EL1 is sufficient to induce a photobiomodulation effect in a human.

The second electro-optical element EL2 may be arranged to emit light in the visible spectrum. Without being restricted in use or form such second electro-optical element EL2 can be a directed or non-directed light emitting device, for direct or indirect illumination or information display purposes, and for direct or indirect irradiation of a user, in particular a user's eyes. Examples can be luminaires, screen devices, mobile devices etc, which may be used in daily context for a user. In particular, such devices may be used for illumination or display purposes and to that purpose emit light in the visible range, indicated in Figure 3 as having a principal emission in the spectral range of 400-700 nm. Furthermore, for illumination or display purposes, the device may comprise several sub sources, for instance a range of light sources in various spectral subranges, in particular, to provide white light as further discussed here below.

Device 100 may further be provided with a driver circuit 110 to drive the first electro-optical element EL1 with a pulse-width, frequency, and radiation level, such that the first electro-optical element EL1 achieves a peak irradiation intensity above 0.1 mW/cm² , preferably above 0.4 mW/cm² , more preferably above 1.0 mW/cm² but an average irradiation intensity less than 10 mW/cm² on the user, as also described in WO2020/119965.

According to an aspect of the present disclosure, a control circuit 120 is arranged to generate an output to the driver circuit 110 to make an adjustment to the first electro-optical element EL1, in response to an automatically generated signal related to usage. Such a signal can be received by control circuit 130 by many implementations, of which the disclosure illustrates some exemplary forms without limiting the scope of potential applications. For the second electro-optical element EL2, the same driver circuit or a separate driver circuit may be used.

For instance, the signal related to usage may generated in accordance with an estimated use time per day of the electro-optical device 100. To this end a 'watch dog' circuit may be registering, as further illustrated in Figure 2, a 'screen time' indicative of user exposer to the device, when a PBM status is active. Based on the screen time indication, a total exposure time may be estimated, which controls a timer 135 that controls the function of the circuit 110.

Usage may already be registered by simply activating the device 100, by a user, to use it for its primary purpose, i.e. to activate the second electro-optical element EL2.

A signal related to usage may also be derived, or a more complex parameter derived from various systems provided in device 100, such as a facial recognition circuit or the like, further illustrated in the below examples.

It may also include an estimated dose based on a proximity detection unit that is programmed to output a distance parameter d of the user; wherein the signal related to usage is generated if a predetermined estimated dose level is exceeded, based on the outputted average distance parameter as further illustrated in Figure 2.

In more detail, Figure 2 shows a further detailed flow diagram of a control circuit 130 in response to an automatically generated signal related to usage. The diagram provides a signal for usage passed on a proximity detection. Such a detection is useful, since the first electro-optical element EL1 is preferably active when a suitable dose can be irradiated, that provides the PBM effect.

Thus, an exemplary form of a usage signal that is generated by control circuit 130 that provides an adjustment signal to driver circuit 110 discussed previously. The control circuit 120 combines a proximity detection with a detected 'on-period', in particular, the signal related to usage is generated if a predetermined maximum on-period is exceeded. The on-period can be detected by a timer 135, that starts a timer when proximity conditions are met.

The timer (or another clock) may additionally serve to indicate the time during a day. It is possible that PBM effects are most efficacious when delivered during daylight hours from the user's perspective. In this case, it may be advantageous for the PBM device to query the timer or clock in the local time-zone and determine whether daylight hours are currently in effect, or not. If not, it may be advantageous, especially from an energy savings point of view, to shut off or disable the PBM device during non-daylight hours.

In the illustrated example, such a condition may be that a user's face is detected within a distance d of 50 cm, or more particular, within a distance ranging between 10 and 50 cm. The lower limit may be determined by a safety aspect, in particular eye safety, further discussed below. The upper limit may be determined by a determined minimum irradiation level, in order to enhance the PBM effect, which will be diminished when irradiation levels are too low.

The on-period may be also detected by other means for example, a screen time parameter, that registers a total usage of screen time for a mobile phone user, further discussed below. For example, the signal related to usage is generated in accordance with an estimated use time per day of the electro-optical device. Another example may include a control circuit that is programmed to provide the signal related to usage as derived from a history table of use device use times.

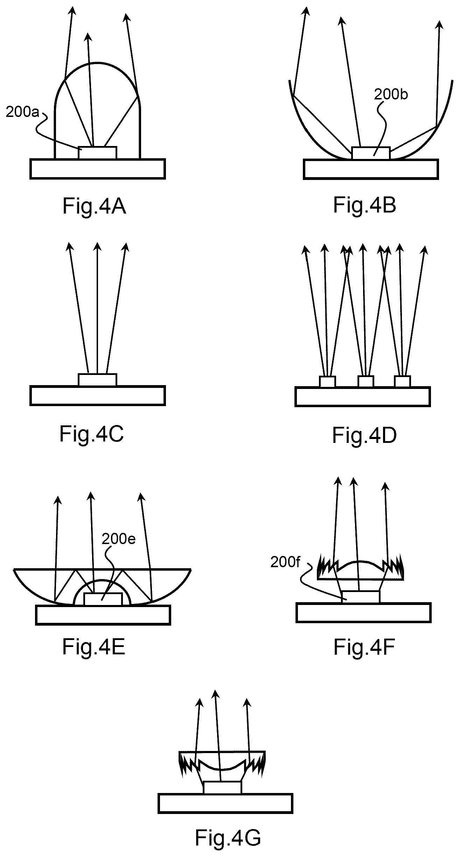

Figures 4a-4g show various embodiments for achieving directed radiation from the first electro-optical element EL1. In particular, there is shown optics to direct the PBM radiation towards the user during operation. By way of example these optics can be used.

The radiation emitted by the first electro-optical element EL1 may be directed by optics so that the radiation is emitted from the device 100 having a radiation pattern with a full-width-at-half-power angle of less than 2x60 degrees, preferably less than 2x50 degrees, and more preferably less than 2x25 degrees.

A wider radiation pattern provides a disadvantage of generating too much radiation that is not directly targeted at a user, so that radiation is wasted and cannot be used to induce a PBM effect. Thus in an optimal sense, the first electro-optical elements EL1 are provided to only irradiate a user with direct radiation impinging on the user's skin, which may be any skin (e.g. in a bathroom environment).

The optics may be refractive (figure 4A) and/or reflective (figure 4B), or a combination thereof, and may be corresponding to electro-optical elements of suitable kinds, in particular LEDs or VCSEL arrays or combinations thereof. Combined forms can use a total internal reflection optics (figure 4E) in combination with lenses, which may be Fresnel type lenses (figures 4F and 4G) of per se known types. Diffractive optics are also options.

VCSEL arrays (vertical-cavity surface emitting laser arrays) are efficient, like LEDs, and can offer even narrower radiation patterns as schematically illustrated in figures 4C and 4D. Also, multi-mode and/or multi-beam VCSEL arrays can be combined with optical elements to slightly diffuse the beams and avoid the laser safety issues associated with high-power single laser beams. A commercially available VCSEL array product, APS6401010002 from II-VI, Inc., delivers 2.2 W typical with a full width beam of 25 degrees at a forward current of 3 A. This particular product is for 940 nm (InGaAs), but a 850 nm (GaAs) version is possible in the current state of the art, and could be expected to have similar performance characteristics. This solution would have about 5 times the irradiated intensity of the LED solution described in the previous paragraph. Also, it may not require a lens element for focusing the emitted beam.

In cases laser devices are used for PBM, it is preferable to avoid any eye safety issues that lasers can trigger. One way to do this is to include an optical diffusor element (between the EELD or VCSEL emitter and the user's eye) that breaks up the coherence of the laser and increases the effect source size, reducing eye safety hazard and allowing one to avoid the requirement of laser-based eye safety ratings for the PBM device.

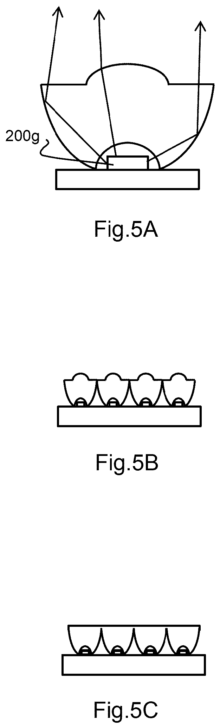

Figures 5a-5c show further embodiments for achieving directed radiation. In the disclosed embodiment in Figure 5A, a LED device 200g may be embedded in lens forming optics, that may be partly shaped to reflect light in forward direction using the principle of total internal reflection (TIR).

For obtaining working depth wherein the PBM effect is kept in the boundaries of having at least a peak irradiation intensity above 0.1 mW/cm² , preferably above 0.4 mW/cm² , more preferably above 1.0 mW/cm² , such optics can require a substantial height e.g. of more than 3 mm.

To reduce the height of the optics, as an alternative, the optics may be designed as microlens array, wherein at a focal centre of each microlens a 'micro'LED is located, which can be suitably achieved by configuring an emitting LED surface in a patterned form of active micro areas and thereby limiting the height of the optics, while at the same time achieving a substantial working depth wherein the PBM effect is still active. These microlens arrays can be of any suitable shape e.g. of the type shown in Figures 5B or 5C.

In another example, the LED may be a single commercially available LED, such as the Luxeon IR Compact L1IZ-A850000000000. The LED may be mounted into a personal device, such as a mobile phone, along with a lens element, as described above, to provide a radiation pattern with a FAHP of 50 (2x25) degrees. A driver circuit 110 is provided within the phone to be able to supply sufficient drive current to achieve a PBM effect.-For example, the LED may be driven at a peak current of 1.5 A in a 1 ms pulse at 10% duty factor (100 Hz) to achieve a peak radiated power output of about 1.9 W at 850 nm. If the lens element is able to collect 80% of this primary radiation, the delivered maximum intensity may be approximately 1.5 W within a target area of 30 cm diameter, or 2.1 mW/cm². The minimum intensity (at the edges of the radiation pattern, at the half-intensity angle) may be 1.1 mW/cm². For 3 hrs of screen time, the delivered dose to the user may thus range between 1.1 to 2.2 J/cm². The total current delivered to the LED may be 1500 mA over 3 hrs at 10% DF, or a total of 450 mAh, which is approximately 15% of the total charge of a typical large smartphone battery (3000 mAh), which should be acceptable to many mobile device OEMs given the expected, never-before-possible health benefits of integrated PBM capability.

Efficiency may be improved by further focusing the radiation, i.e., narrower FAHP, increasing the optical efficiency of the lens, or the efficiency of the LED itself. Electric current to the PBM device EL1 may be provided by electronic driver chips, such as those developed for the mobile phone LED "flash" function. For example, the Flash LED Driver KTD2681 from Kinetic Technologies can deliver up to 1.5 A in programmable, pulsed modes.

To achieve low-profile designs so-called mini-LED and micro-LED can be utilized as shown in Figures 5B and 5C. Mini-LEDs (typically 250 um or less in chip dimensions) and micro-LEDs (typically less than 50 um chip dimensions) have been of interest lately in applications such as displays. Since optical elements scale with chip size, the overall size of a lens or reflector element may be reduced by replacing a high-power LED chip 200 with a mini- or micro-LED array (of the same total area in summation). Ideally, the chip height is reduced proportionally, which may be achieved by thinning (or even removing) the LED chip substrate.

Micro-LED based displays and illumination systems have become of interest in recent years. For displays, unlike using conventional LCD or OLED stacks, micro-LEDs offer the possibility to build up display pixels using a very small amount of semiconductor material, compared to the surface area of the display itself. This means there is room to include additional components into the display, either between pixels or within a pixel. For example, red, green, and blue micro-LEDs included into a pixel might also include a NIR emitter, either NIR micro-LED or VCSEL. This way, NIR emission can be integrated seamlessly into the display and used to provide PBM effects when desired.

An example of total height reduction using a dome-lens mini-LED array approach is illustrated in Fig. 5B. As comparative example, the commercially available IR LED L1I0-Axxx050000000 from Lumileds has a FAHP of 2x25 = 50 degrees and a total height of 3.4 mm, of which 2.6 mm is attributed to the lens and 0.8 mm to the chip and submount. The chip in this LED product is 1 x 1 mm². Breaking up the LED chip into 16 mini-LEDs of 250 x 250 um² each, one is entitled to shrink the height of the lens to ¼ of its original value, or 0.65 mm, while maintain the same optical efficiency. This assumes the LED chip height is reduced as well (for example, by substrate thinning). The total thickness of the mini-LED based package in this case is 0.8 + 0.65 = 1.45 mm, and the optical performance and FAHP can be similar as for the comparative example.

As an alternative embodiment, an example of a TIR-lens mini-LED array is shown in Fig. 5C. As for the dome lens case described above, breaking the power LED chip up into a 4x4 array of mini-LEDs, one is entitled to reduce the height of the TIR lens element by a factor of four. When the chip width becomes so narrow that the side emission dominates over top emission, it may be permissible to eliminate the top surface dome elements to simplify the design and reduce profile further, while still achieving high optical efficiency (see "alternate" design in the figure).

Mini- and micro-LED devices can be fabricated using conventional LED fabrication techniques and be assembled with conventional chip-handling techniques in some cases. When the devices become very small or it is necessary to handle very many, then advanced techniques such as mass-transfer may be utilized. Mass-transfer techniques are known in the art and include elastomer pick-and-place, laser transfer, and electrostatic pick-and-place, among others.

In a particular example, a PBM device EL1 may comprise a single high-power IR LED that is mounted on a printed circuit board (PCB) within the mobile phone device, such that the LED emission is able to be directed at the user's face when the phone is being used. A lens element between the device and the user is included in order to focus the LED emitted radiation and increase the delivered irradiated intensity.

For example, a target radiation pattern may be a full-angle at half-power (FAHP) of approximately 50 degrees. Such a radiation pattern may ensure that most of the delivered infrared radiation from the PBM device EL1 is delivered to a useful part of the target subject, when the subject's face is 30 cm (a typical distance) away from the phone. Also, it ensures that the vast majority of the user's (exposed) face may experience the PBM radiation, and not just some small portion of the face.

It may be preferable that the lens element is a planar optic, such as a Fresnel lens, rather than a standard refractive lens element, in order to conserve space (thickness) within the mobile phone construction.

As described above, the PBM device may be pulsed in order to provide the required intensity levels, while easing overall thermal management and energy use. If the PBM device is pulsed, the minimum pulse length should be at least one ms, to ensure an effective PBM stimulus. Also, if the PBM device is pulsed, the repetition frequency should be such that operation of the device does not cause image flicker which can be picked up by imaging devices in the vicinity of use, including the mobile phone camera itself.

For example, it may be preferable to have a repetition rate for pulses of at least 100 Hertz or more as disclosed in US Provisional application 63166331.

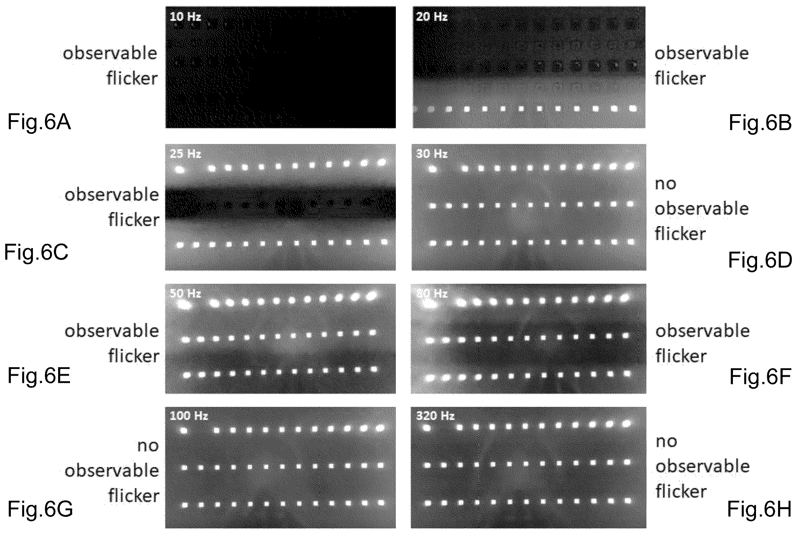

Figures 6a-6h show a number of driving frequencies wherein image flicker is prevented. The illustrated examples show that around 80 Hz flicker may still be observable, whereas at pulse frequencies above 80 Hz, such image flicker can be prevented.

The images shown are video screen-shots generated from a smartphone camera of an NIR LED array pulsed at variable frequencies from 10 to 320 Hz at ambient intensity levels high enough to be observed; image A at 10 Hz, B at 20 Hz, C at 25 Hz, D at 30 Hz, E at 50 Hz, F at 80 Hz, G at 100 Hz, and H at 320 Hz. Image flicker is observed at 10 Hz, 20 Hz , 25 Hz, 50 Hz, and 80 Hz. The image flicker shows up as horizontal stripes in the screen shots. It is notable that image flicker is not observable at 30 Hz, which corresponds to the frame rate of the smartphone camera used for the imaging in Figures 6a-h. It is also notable that no observable image flicker is observed at 100 Hz or higher frequencies.

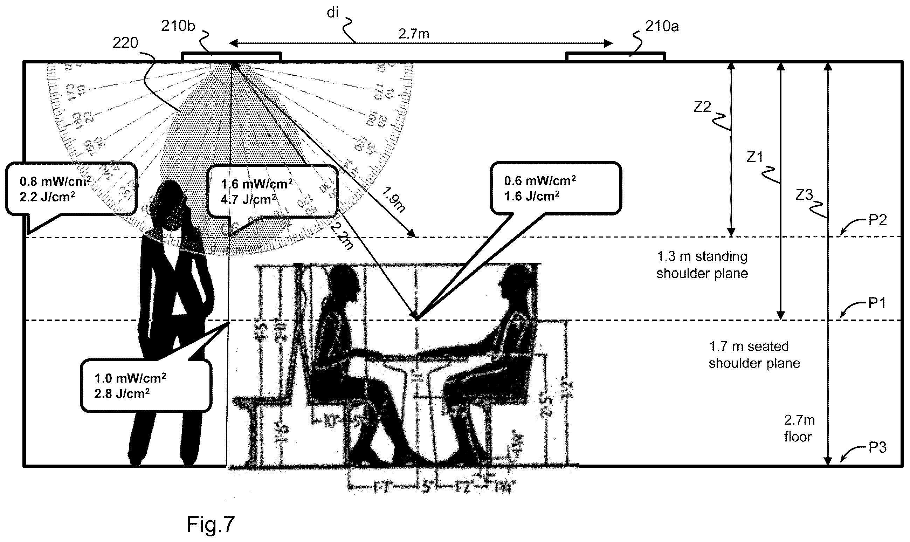

Figure 7 shows a schematic irradiation pattern of the first electro-optical element EL1 for a luminaire 210 as an example of an illumination device installed for illumination purposes in a direct vicinity of the user. The luminaire 210 is thus enhanced with a first electro-optical element EL1 for providing the PBM effect, at the same time comprising a light source including the second electro-optical element EL2 which is adapted to emit white light suitable for general illumination, e.g. wherein the light source is adapted to emit at least 250 lumens, preferably at least 1000 lumens, more preferably at least 2000 lumens when operating. Exemplary forms as disclosed show PBM capability integrated into troffer luminaires 210 typically used for general illumination.

In one embodiment, a typical office environment is considered. Typical ceiling height Z3 is 2.7 m. Typical height of the shoulders for a seated person is 100 cm, providing a target distance Z1 for PBM action at 170 cm below the troffer panel, determining a first target "irradiation plane" P1. A second target irradiation plane P2 is at shoulder height for a person standing below the troffer luminaire 210. Typical height of the shoulders for a standing person is 140 cm, providing a target distance Z2 for PBM action at 130 cm below the troffer panel for the second target irradiation plane P2. A typical "2x4" panel delivers about 3000 lumens and can be spaced a ratio of between 1:1 to 2:1 with respect to the ceiling height Z3, or between 2.7 to 5.4 m.

In a particular example, a PBM device EL1 comprising 28 high-power IR LEDs is mounted on one or more printed circuit boards (PCBs) within a troffer panel, such that the LED emission is directed downward. A lens element between the device and the user is included in order to focus the LED emitted radiation and increase the delivered irradiated intensity. For example, a target radiation pattern may be a full-angle at half-power (FAHP) of approximately 90 degrees. As described previously, the PBM device may be pulsed in order to provide the required intensity levels, while easing overall thermal management. If the PBM device is pulsed, the repetition frequency should be such that operation of the device does not cause image flicker which can be picked up by imaging devices in the vicinity of use. For example, it may be preferable to have a repetition rate for pulses of at least 100 Hertz or more.

For example, the LEDs may be commercially available LEDs, such as the Luxeon IR LED L1IZ-A850090000000. The LEDs may be mounted into the troffer, and come with an integrated lens to provide a radiation pattern with a FAHP of 90 degrees. A driver circuit 110 is provided within the panel to be able to supply sufficient drive current to achieve a PBM effect. For example, the LEDs may be driven at a peak current of 3.0 A in a 1 ms pulse at 10% duty factor (100 Hz) to achieve a peak radiated power output of about 3.1 W each at 850 nm.

Multiple troffer panels may be used such that they are spaced at 2.7 m, the same distance dᵢ as the ceiling height Z3. At the first target irradiation plane P1, the delivered peak intensity may be approximately 1.0 mW/cm² directly beneath one of the troffers 210. Directly in-between the troffers 210, the peak irradiated intensity may less, e.g. about 0.6 mW/cm². At the second target irradiation plane P2, the delivered peak intensity may be approximately 1.6 mW/cm² directly beneath one of the troffers. Directly in-between the troffers, the peak irradiated intensity may be less, e.g. about 0.8 mW/cm². For a target subject experiencing 8 hrs of interaction within the beam radiation pattern under the panel, the delivered dose may range between 1.6 to 4.7 J/cm². Assuming the IR LEDs are constantly pulsing during an 8 hour period, the average power dissipated by the LEDs during this period may be about 30 W, likely less than the power consumption of the troffer panel for illumination purposes.

As described above, the IR LEDs can be more efficiently operated by only operating them when an 'on' condition is determined, such as when the presence of a target subject is detected by an associated sensor device, which may be incorporated into the troffer or may be an external device in communication with the IR LEDs, for example a remote control.

Also, it is preferable to have the IR LEDs on only during a limited time of day, such as an 8-hour period within a time window typical for daylight hours. When an 'on' condition is determined, rather than turn on abruptly, the IR LEDs might be ramped up (either in amplitude, or duty factor) to their target operating level slowly, over several seconds or even minutes, so as not to disturb people or devices in the vicinity sensitive to sudden changes in level of ambient red and/or IR radiation.

For wider spacing between troffer panels, it is possible to utilize a so-called "batwing" radiation pattern optics for the NIR LEDs in order to deliver a more uniform irradiated intensity throughout the target irradiation plane. Batwing radiation pattern optics are well known in the art and may be coupled with NIR devices to achieve the described effect.

The calculated irradiated intensity levels discussed above represent direct-only (single-pass) IR radiation. In actuality, reflections off surfaces (e.g., walls, ceiling, desks, etc.) may serve to enhance peak IR irradiation levels and increase PBM effect efficacy, or reduce required power levels to achieve a PBM effect.

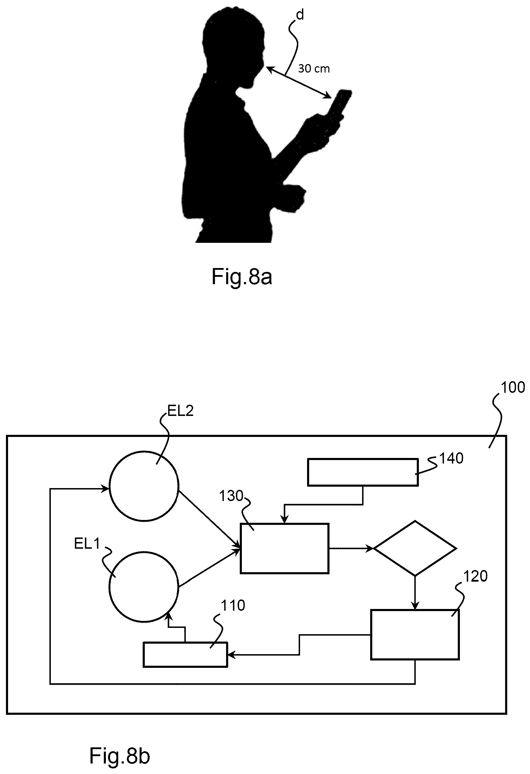

Figure 8a shows a schematic irradiation pattern for a handheld device, as measured by Alfaitouri et al. The Effect of Posture and Duration of Smartphone Usage on Neck Flexion Angle, Proceedings of the Human Factors and Ergonomics Society 2019, Annual meeting, 962-966.

According to an aspect of the present disclosure, a control circuit 120 that makes an adjustment to the first electro-optical element EL1, in response to an automatically generated signal related to usage. The adjustment can be an off-switching of the electro-optical element EL1, or a tempering, e.g. by lowering the radiation level.

The control circuit 120 may further be programmed to ensure that a predetermined cumulative off-period is longer than 8, preferably longer than 12 hours so that a body may suitably adapt to the PBM effect without overexposure by unintended restarting of an PBM irradiation programme. For example, an adjustment circuit may control that a delivered dose over 1 hour or longer at an average distance of between 0.1 and 5m from the first light source is between 0.01 and 50 J/cm², and preferably between 0.1 and 10 J/cm².

In addition to a usage control circuit 130 to control safety with respect to the present disclosure from a PBM dose point-of-view, it is also important to care for eye safety. In accordance with IEC 62471, Photobiological safety of lamps and lamp systems*,* to protect against retinal thermal injury, the source average radiance is managed so that the retinal thermal hazard limit, L_IR, is not exceeded.

The handheld device may be any mobile device, including mobile phones but also mobile screen devices such as tablets, game devices, AR/VR headsets, watches, smart glasses, etc. The mobile devices may comprise a second electro-optical element EL2 suitable for providing visible light. Under conditions with weak or no visual stimulus to the eye, the L_IR can be fairly low. For example, for a 850 nm IR emitter and worst-case assumptions regarding source angular subtense, L_IR is 60000 W/m²/sr. Conservatively, if one considers a 1 x 1 mm² power IR LED chip so that all radiation exits the top surface in a Lambertian emission pattern, L_IR can be exceeded when the optical power of the LED chip exceeds 60000 x p x 1E-6 / (R(λ) = 0.5 for 850 nm) ~ 0.38 W average power. For a 10% duty factor situation, the peak power per LED is preferably kept less than 3.8 W.

The retinal thermal hazard limit can be raised considerably if the eye is subjected to, simultaneously with IR radiation exposure, visual stimulus above 10 cd/m², which is easily met by most illumination and display systems (which typically emit 100 cd/m² or more). Under these conditions, the L_IR limit according to IEC 62471 under worst case assumptions increases to 281171 W/m²/sr.

As above, if one conservatively considers a 1 x 1 mm² power IR LED chip so that all radiation exits the top surface in a Lambertian emission pattern, L_IR can be exceeded when the optical power of the LED chip exceeds 281171 x p x 1E-6 / (R(λ) = 0.5 for 850 nm) ~ 1.77 W average power. For a 10% duty factor situation, the peak power should be kept less than 17.7 W. Since the retinal thermal hazard limit is considerably higher when visual stimulus above 10 cd/m² is present along with IR radiation, the visual stimulation is included in embodiments of the present invention.

Also, in cases wherein the visual stimulation is not present, it may be preferable to reduce the power and or turn off the IR radiation source to ensure retinal thermal hazard limit is not exceeded.

Thus, in a preferred example embodiment, a device which radiates directly in a user's eye is preferably provided with an eye detection or face recognition circuit 140 (see figure 8b).

The infrared radiation hazard exposure limit, E_IR, for the human eye is driven by irradiance. According to IEC 62471, the limit is 10 mW/cm² average irradiance within 780 to 3000 nm. For an emitter operating at 10% duty factor, the limit in terms of peak irradiance is 100 mW/cm².

As explained previously with relation to Figure 2, usage control circuit 130 may include a proximity detection unit programmed to output a distance parameter d of the user; and wherein the signal related to usage is generated if a predetermined estimated dose level is exceeded, based on the outputted average distance parameter. In particular for mobile phones, the signal related to usage may be generated in accordance with an estimated use time per day of the electro-optical device 100, which can e.g. be derived from a history table of use device use times.

Control circuit 130 may further comprise a dose calculation unit that provides a calculated exposure schedule for the driver circuit 110 and wherein a signal related to usage is generated when a predetermined dose is exceeded.

The first electro-optical element EL1 may be arranged to emit only when the second electro-optical element EL2 is emitting with a radiation intensity above 10cd/m² . Eye detection circuit 140 may provide instructions to control circuit 130 to this effect, which may suitably a radiation level of the second electro-optical element EL2. This may suitably trigger a pupil response and provide a means to irradiate in a safe condition.

Accordingly device 100 may comprise a controller 130, 140, 120 to detect that a user (visually) contacts the first electro-optical element EL1 and in response performs the action of generating the signal related to usage, to adjust a level of irradiation of the first electro-optical element EL1.

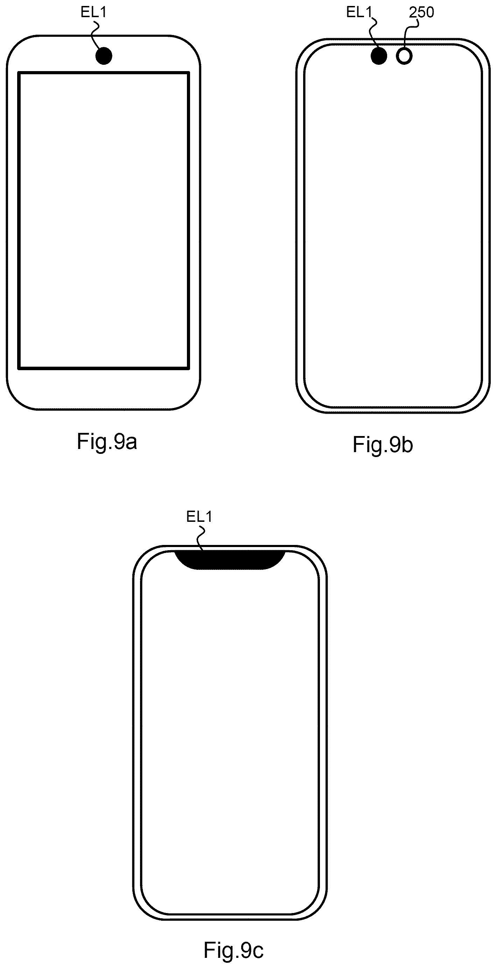

Figures 9a-9c show embodiments in which the electro-optical device is incorporated in a handheld device, such as a smartphone or tablet.

Since the distance between such a handheld device and the user is predictable (e.g. at 25 cm or 30 cm), the required radiation intensity can be achieved reliably even without any sensor. The radiation emitted by the first electro-optical element EL1 may be directed so that the radiation is emitted from the device having a radiation pattern with a full-width-at-half-power angle of less than 2x60 degrees, preferably less than 2x50 degrees, and more preferably less than 2x25 degrees. Advantageously, the first electro-optical element EL1 may be arranged in an area of the device that also contains a facial recognition sensor or facial detection sensor, as shown in Fig. 9c. This can further improve dose control and other control functionalities of the device.

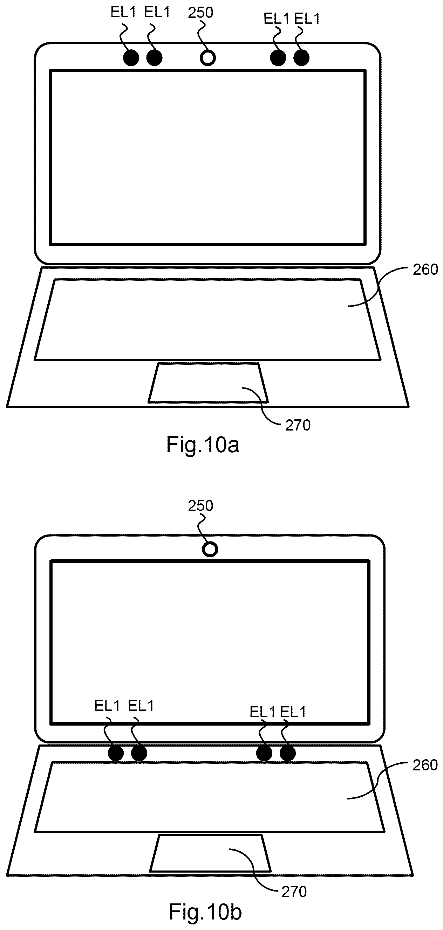

Figs. 10a and 10b show embodiments in which the electro-optical device is incorporated in a computer, such as a laptop computer. Also in this case, the predictable distance between the device and the user allows the desired radiation intensity to be reliably achieved. The first electro-optical element EL1 may be arranged at any place of the computer, such as above the screen (see e.g. Fig. 10a) or next to the keyboard 260 (see e.g. Fig. 10b). The radiation emitted by the first electro-optical element may be directed so that the radiation is emitted from the device having a radiation pattern with a full-width-at-half-power angle of less than 2x25 degrees, preferably less than 2x15 degrees.

In the embodiments of Figures 9a-9c and 10a, 10b, the electro-optical element EL1 may alternatively be integrated in the display, e.g. using micro-LEDs discussed above.

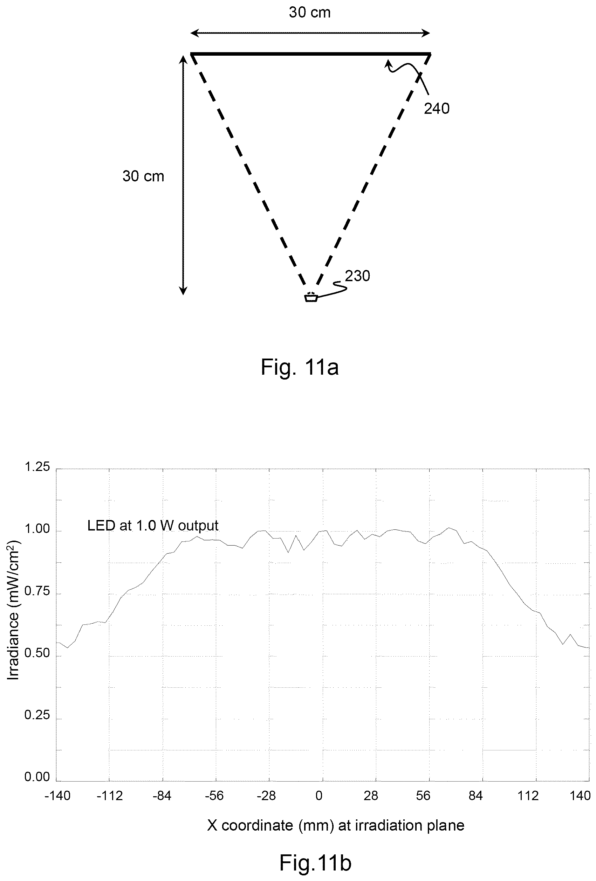

Figure 11a shows a typical irradiation plane 240 for providing PBM radiation to a person. Although in the figure, typical distance and plane dimensions are about 30 cm, this is only exemplary for a range, that may be in the order of 10 cm or more, e.g. 50 cm, or even 5000 cm, depending on the application (tv screen etc). The exposure area within an irradiation plane 240 is typically limited to a human lateral dimension, such as face with or body width, and may typically not exceed 50 cm.

The device or apparatus of the present disclosure contains a dose monitoring system, such as a clock and distance sensor in order to calculate a cumulative dose. In addition, the device may include a proximity or other sensor (e.g., face detection) to determine an 'on condition'. The dose monitoring means the device can determine an 'off condition' as well. The device may contain these and other sensors and timers to provide a feedback loop for precise dose control and even distribution of power throughout the time duration.

Irradiance is distance dependent. Embodiments of the present disclosure include controls that serve as safety mechanisms for the human eye. For example, a proximity sensor or face-recognition system can determine the distance d of a user's eye from the PBM radiation source. If the combination of PBM source power and distance of the user's eye would result in exceeding E_IR, the PBM source power shall be turned down or even off.

As an example, the inventors have had designed a Fresnel lens system to work with a commercially available IR LED, Lumileds part no. L1IZ-A850000000000. The LED inserts into a cavity in the back of the lens and parabolic side-surfaces in combination with a Fresnel lens top surface provide a focusing effect in a compact design, such that the total height from the top of the lens to the bottom of the LED is 3 mm. This lens has approximately 50% beam efficiency (into a 2x25 degree beam) and is able to produce about 1 mW/cm² irradiance at 30 cm from a single IR LED emitting 1 Watt of optical power. If the source is driven cw, the LED power must be turned down or off when the users eye closes in on 3 cm distance from the LED, since the irradiance can be 100x higher than at 30 cm, or ~ 100 mW/cm² (the E_IR limit), according to the inverse square law of radiation.

As another example considering the same lens arrangement, if a single LED is operated at 10% duty factor, but at a higher peak optical output, such as 5 W (giving a 0.5 mW/cm² average irradiance), then the power should be turned down when the eye reaches a distance of 2.12 cm from the LED source.

In cases of multiple LEDs with radiation patterns that overlap, power may need to be reduced at distances even further from the device than described immediately above. For example, a tablet including 4 LEDs running at 5W each at 10% duty factor can exceed an irradiance of 100 mW/cm² at a distances closer than 8.5 cm to plane containing the LED array. In this case, the PBM tablet device should turn down or turn off power to the LEDs when the user's eye approaches this distance, so that the E_IR limit is not exceeded.

While the invention is illustrated and described in detail in the drawings and foregoing description, such illustration and description are to be considered illustrative or exemplary and not restrictive; the invention is not limited to the disclosed embodiments. Furthermore, the skilled person will appreciate that the term test used herein is not limited to a specified physical designs, but may encompass all kinds of structures that may interconnect with the chip die.

Furthermore, the invention is not limited to specific types of applications but may be used in applications such as Automotive interiors; aviation interiors; Industrial lighting; Outdoor lighting; Residential lighting; Commercial/architectural lighting; Project- Table top & overhead; Desktop Monitors; Large displays, TV's; Projectors, pico and highly portable; public transportation displays; Industrial purposes e.g. (O)Led based lighting/ illumination for processing purposes (e.g. curing), wearables, computer accessories, (tech) gadgets, 3-phase rail accessories, etc.

The second light may be a light source capable of white light, that is, a light with an emission spectrum having a chromaticity near the so-called blackbody line in chromaticity space, i.e. composed of a spectral composition of wavelengths in the range illustrated in Figure 3.

As defined in ANSI C78.377, white light includes chromaticities along the blackbody line and within +/- 0.006 of it in terms of Duv, a distance from the blackbody line in u'v' chromaticity space. ANSI C78.377 defines white for color temperatures between 2200 and 6500K, but the second light may provide color temperatures outside this range. The second light may also produce so-called tinted white light, which is light that has a chromaticity near but outside the area specified by the ANSI C78.377 +/- 0.0006 Duv limit around the blackbody line in u'v' chromaticity space.

The second light may be from a display, such as a television, monitor, laptop, tablet, notebook, or phone device. The second light preferably emits with a luminance greater than 1 cd/m², more preferably greater than 10 cd/m². At these luminance levels and above, pupil size adaptation comes into play, which reduces the risk of eye safety hazard for exposure to infrared radiation used for PBM. A fully dark-adapted pupil of the human eye is approximately 7 mm in diameter. In the presence of a white light source with luminance of 1 cd/m², the pupil shrinks to about 5 mm. At 10 cd/m², it shrinks to about 4 mm in diameter. (Watson, A. B., & Yellott, J. I. (2012). A unified formula for light-adapted pupil size. Journal of Vision, 12(10) 12, 1-16)

Smartphone users have a typical posture (Figure 8a), and the distance d between the phone and the face is well known and is approximately 30 cm. In addition, the time people spend using their phone every day is well known (typically >3h). Using this information, and the target ranges for PBM irradiation intensity and doses as determined by the inventors, we can describe a practical PBM device for integration into a smartphone.

People interact with their mobile phones in a very predictable manner, as described above. Furthermore, many mobile phones have mechanisms for monitoring the duration of human interaction with the device, so-called "screen time". The average screen time for a mobile phone user nowadays is typically 3 hours per day.

Knowledge of the distance d of the user's face from the phone, and the total amount of screen time, allows us to calculate a meaningful PBM dose for the user of the phone to provide health benefits, for example, in case the user does not have access to natural solar radiation. In this embodiment, a PBM device EL1 is included in the mobile phone facing towards the user. The PBM device is capable of delivering IR radiation to a user's face at an irradiated intensity level greater than 0.1 mW/cm², preferably greater than 0.4 mW/cm², and more preferably greater than 1.0 mW/cm².

The PBM device delivers the IR radiation during the time that the user's face is interacting with the phone, as monitored by the phone, but may be turned off when the user is not interacting with the phone, to save energy.

For the typical use case of three hours per day of screen time, and an irradiated intensity level greater than 1 mW/cm², the achieved dose is greater than 10 J/cm² for continuous wave (cw) operation of the PBM device. However, as determined by the inventors, doses as low as 1 J/cm² could have a positive PBM effect. Thus, the PBM device could be operated in a pulsed mode at 10% duty factor (DF) and deliver > 1 J/cm², preferably >4 J/cm².

Alternatively, higher intensities are of course possible but come at the expense of battery drain on the phone. There is also the risk of overdosing the target subject. Overdosing has been reported in scientific literature for in vitro studies, animal models, and local treatments on humans, and depends on wavelength, but hard numbers for systemic PBM remain elusive.

The mobile phone may include circuitry and/or sensors to monitor the on-time of the PBM device, similarly to the software-sensor system that puts the phone in stand-by mode, and can shut down the PBM device if a target PBM dose level has been achieved or if battery conservation for the phone is required.

In line with the standard practice, a skilled person would understand that transmission could be turned off by switching to a minimum non-zero power value, e.g. within a tolerance. In the context of the present invention, the terms "shut off" and "shut down" would similarly be understood as also covering non-zero small power values, e.g. at a level at which the electromagnetic radiation by the electro-optical element EL1 can barely induce any PBM response.

The above detailed example for a smartphone similarly applies to applications such as a tablet and e-reader. The present invention can also be applied to other devices, such as laptop computers, rear-of-seat displays in aircraft and buses, displays in fitness devices like treadmills and spinning bikes, and similar displays. In these applications, a typical distance between the screen and user is about 50 cm. In this case, in order to meet the PBM dose requirements and efficiently deliver radiation, a LED with a narrower FAHP compared to the above example would be required, such as 30 degrees.

In cases where this is difficult to achieve due to space requirements for the focusing lens, another option is to increase the number of LEDs. For example, using four (instead of one) LEDs used for the detailed example above will be able to deliver meaningful PBM doses at the same screen time, for these applications. The extra power requirement is less of a concern in these cases, compared to a smartphone, because of availability of larger batteries and/or external power supplies for these devices.

Similar configurations could be employed for PBM devices in computer keyboards, or in controls for video games, wherein optics are included to direct the PBM radiation towards the user during operation. Also, this configuration could apply to drivers of cars, by including the PBM devices in the steering wheel or dashboard, or dome in the car ceiling (or above passengers in an airplane), or users of fitness equipment.

NIR light/PBM is also linked to positive effects on the human eye. In combination with smartphone use, which is often claimed to have negative effects on vision, this could be a new unique selling point. Reference is made to Boris T. Ivandic and Tomislav Ivandic.Photomedicine and Laser Surgery.Jun 2008.241-245; Goo H, Kim H, Ahn J, Cho KJ Effects of Low-level Light Therapy at 740 nm on Dry Eye Disease In Vivo. Medical Lasers 2019;8:50-58; Geneva, Ivayla I. Photobiomodulation for the treatment of retinal diseases: a review. International journal of ophthalmology vol. 9,1 145-52. 18 Jan. 2016.

When viewed directly, NIR devices can still be faintly perceptible to the human eye. For example, a dim and dark-red glow may be observed when viewing the emitting surface of NIR LED. In some cases, this appearance may not be desirable. In such cases, band-pass or long-pass filters materials may be placed between the emitter and the viewer, for example integrated into the housing of smartphone, tablet, laptop, or any of the other devices useable by the invention. These materials transmit the majority of NIR radiation, but block radiation in the visible spectrum, e.g., less than 700 nm, or less than 780 nm. Such materials are commercially available today, such as the Color IR transmission sheet / White Color IR transmission sheet family of products from Tokai Optical Co., Ltd. When employed between the NIR device and the user, such materials provide that the device can deliver its intended PBM effect, while not exhibiting any perceptible glow to the human eye during operation.

### Television Display

Further extending the PBM-integrated display concept, it is conceivable to include PBM in television (TV) displays. The average viewing distance for a TV depends on its size. A rough rule-of-thumb is a seating distance of two times the screen diagonal. Using this rule-of-thumb, we can calculate the number of LEDs or VCSEL devices required to deliver a meaningful PBM effect (in this case > 1 mW/cm² peak irradiated intensity) as described above, as shown in the table below:

| 10% DF Pulsed Operation | **Diagonal** | | **Viewing distance** | **No. LEDs** | **No. VCSELs** |
|---|---|---|---|---|---|
| | **in** | **cm** | **cm** | **50-deg FAHP** | **25-deg FAHP** |
| | | | | | |
| Smartphone | 5 | 13 | 25 | 1 | < 1 |
| Tablet | 11 | 28 | 56 | 4 | 1 |
| TVs | 32 | 81 | 163 | 36 | 8 |
| | 40 | 102 | 203 | 64 | 13 |
| | 55 | 140 | 279 | 121 | 25 |
| | 65 | 165 | 330 | 169 | 34 |
| | 75 | 191 | 381 | 225 | 46 |
| | 85 | 216 | 432 | 289 | 59 |

The table above is for 10% duty-factor pulsed operation, as described in detail previously. The device count can be reduced (by up to 10x) by operating the devices in continuous wave (cw) mode, which is possible especially for larger displays wherein there is plenty of space for thermal management. Alternatively, a narrower radiation pattern can also reduce the required number of devices, but care should be maintained to achieve the target coverage area in the application, for example, each sitting area in a living room outfitted with a PBM-integrated TV.

### Bathroom Environment

PBM may also be included in beauty and home-care environments, such as in bathroom mirrors or in showers. As in the cases above, the location of the user is fairly well known, and their proximity can be detected (e.g., by a proximity detector, or other proxy such as shower water being turned on). In these applications, however, in contrast to displays, the amount of time the user interacts with the devices may be shorter. For example, the average shower duration is approximately 8 minutes, which is much shorter than typical daily screen time.

In the detailed example for the shower application, the LED may again be a commercially available LED, such as the Luxeon IR Compact L1IZ-A850050000000, which includes an integrated lens which gives a FAHP of 50 degrees. In this case, multiple LEDs will be necessary.

The LEDs may be mounted into a fixture near the showerhead or may be integrated into a dome fixture in the ceiling of the shower cabin. A power supply and driver circuit are coupled to the LEDs to supply sufficient drive current to achieve a PBM effect. In some embodiments, the LED light may be coupled into an optical fiber or bundle-fiber and delivered into the shower, so that the LEDs and electronics may be kept out of the immediate shower area.

Due to the short time duration, it is helpful to drive the LEDs in cw mode in order to achieve the intended dose. For example, five LEDs may be driven each at a continuous current of 1.5 A to achieve a continuous radiation output of about 1.8 W at 850 nm. Assuming the entire optical system collects 80% of this primary radiation, the delivered average intensity will be approximately 1.4 W in the peak of the beam, within a target area of 45 cm diameter at a distance of 50 cm, or 4.6 mW/cm². The minimum intensity (at the edges of the radiation pattern, at the half-intensity angle) will be 2.3 mW/cm². For an 8 minute shower, the delivered dose to the user will thus range between 1.1 to 2.2 J/cm². The power delivered to the LEDs will be approximately 24 W during their "on" time.

The "on" time can be controlled by including a proximity sensor in communication with the LED control electronics, to only allow them to turn on when someone is present in the shower cabin, or coupled to a water flow sensor, to only allow them to turn on when shower water is flowing, or both conditions are satisfied.

Similar calculations as the above apply to a bathroom, or dressing-room, applications, wherein, for example, the LEDs might be integrated into a mirror, behind a mirror, or into lighting luminaires in the walls or ceilings. Proximity sensors can be included in the area to detect the presence of the person, allowing PBM devices to turn on only when someone is present.

Certain sensors may detect the distance d of the subject from the PBM devices, so that dose can be monitored. Thus, the PBM devices may be controlled so that they are only on when they are useful, are targeted to deliver a meaningful dose PBM effect in a reasonable amount of time, and are shut off when they are no longer needed, to avoid an over-dosing situation but also to conserve energy.

Of course, such systems could be installed in any other area of a house, or other built environment for humans, including spas, gyms, workplaces, workstations, etc.

Other variations to the disclosed embodiments can be understood and by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### CLAUSES

1. An electro-optical device (100) capable for providing a photobiomodulation effect in a user of the device (100), comprising:
   at least one electro-optical element (EL1) arranged to emit electromagnetic radiation having a peak emission wavelength between 700-1400 nm;
      a control circuit (120, 130) configured to adjust emission characteristics of the electro-optical element; and
   a driver circuit (110) configured to receive an input from the control circuit (120, 130) and drive the electro-optical element based on the input received from the control circuit (120, 130), using a driving current that is pulsed,
      wherein the control circuit (120, 130) comprises a timer (135) configured to determine an on-period for the electromagnetic radiation emitted by the electro-optical element (EL1), and/or comprises a dose calculation unit configured to determine an accumulated dose of the electromagnetic radiation at the user;
      wherein the control circuit (120, 130) is configured to instruct the driver circuit (110) to shut off the electro-optical element (EL1), if a predetermined maximum on-period is exceeded, and/or if the accumulated dose determined by the dose calculation unit indicates that a predetermined dose of the electromagnetic radiation at the user is exceeded.
2. The electro-optical device (100) according to clause 1, wherein the circuit (120, 130) includes a proximity detection unit (130) configured to determine a distance (d) between the user and the device (100), wherein the control circuit (120, 130) is configured to control the timer and/or the dose calculation unit in dependence on the determined distance (d).
3. The electro-optical device (100) according to clause 1 or 2, wherein the electro-optical device is a handheld device, wherein the driver circuit is configured to drive the electro-optical element with a pulse-width, frequency and radiation level, such that the electro-optical element achieves a peak irradiation intensity above 0.1 mW/cm² on the user, wherein the first electro-optical element is be directed so that the radiation is emitted from the device having a radiation pattern with a full-width-at-half-power angle of less than 2x60 degrees, preferably less than 2x50 degrees, and more preferably less than 2x25 degrees.
4. The electro-optical device (100) according to any of the preceding clauses, wherein the electro-optical device is a computer, wherein the driver circuit is configured to drive the electro-optical element with a pulse-width, frequency and radiation level, such that the electro-optical element achieves a peak irradiation intensity above 0.1 mW/cm² on the user, wherein the radiation emitted by the first electro-optical element is directed so that the radiation is emitted from the device having a radiation pattern with a full-width-at-half-power angle of less than 2x25 degrees, preferably less than 2x15 degrees.
5. The electro-optical device (100) according to clause 1 or 2, further comprising a second electro-optical element (EL2) being an illumination device (210), wherein the driver circuit is configured to drive the electro-optical element with a pulse-width, frequency and radiation level, such that the electro-optical element achieves a peak irradiation intensity above 0.1 mW/cm² on the user, wherein the radiation emitted by the first electro-optical element is directed so that the radiation is emitted from the device having a radiation pattern with a full-width-at-half-power angle of less than 2x25 degrees, preferably less than 2x10 degrees.
6. The electro-optical device (100) according to any of the preceding clauses, further comprising a second electro-optical element (EL2), wherein the first electro-optical element (EL1) is arranged to emit only when the second electro-optical element (EL2) is emitting with a luminance above 10 cd/m² .
7. The electro-optical device (100) according to any one of the preceding clauses, wherein adjusting of the emission characteristics of the first electro-optical element (EL1) by the control circuit (120, 130) involves shutting the first electro-optical element off for a predetermined off-period.
8. The electro-optical device (100) according to clause 7, wherein the predetermined cumulative off-period is longer than 8 hours, preferably longer than 12 hours.
9. The electro-optical device (100) according to any one of the preceding clauses, wherein the peak irradiation intensity at an average distance of between 0.1 and 5m from the first electro-optical element (EL1) is 0.1 mW/cm² or more, preferably between 0.4 and 50 mW/cm², and more preferably between 1 and 15 mW/cm².
10. The electro-optical device (100) according to any one of the preceding clauses, wherein a delivered dose over 1 hour or longer at an average distance of between 0.2 and 5m from the first light source is between 0.01 and 50 J/cm², and preferably between 0.1 and 10 J/cm².
11. The electro-optical device (100) according to any of the preceding clauses, wherein the radiation emitted by the first electro-optical element (EL1) is directed by optics so that the radiation is emitted from the device having a radiation pattern (220) with a full-width-at-half-power angle of less than 2x60 degrees, preferably less than 2x50 degrees, and more preferably less than 2x25 degrees.
12. The electro-optical device (100) according to any of the preceding clauses, wherein the radiation emitted by the first electro-optical element (EL1) has a radiation pattern (220) with a full-width-at-half-power angle of 2x10 degrees or less.
13. The electro-optical device (100) according to any of the preceding clauses, wherein the electro-optical device (100) is a display screen, comprising a first set of micro-LEDs or VCSELs arranged to emit electromagnetic radiation having a peak emission wavelength between 700-1400 nm to function as the electro-element (EL1), and a second set of micro-LEDs to function as display pixels.
14. The electro-optical device (100) according to any of the preceding clauses, wherein the pulse frequency is 100 Hz or higher.
15. The electro-optical device (100) according to any of the preceding clauses, wherein the pulse frequency is 30 Hz or a multiple of 30 Hz, preferably a common multiple of 24 Hz and 30 Hz, such as 120 Hz.

## Claims

1. An apparatus for a display system, capable of providing a photobiomodulation effect in a user of the system, the apparatus comprising:
- at least one electro-optical element (EL1) arranged to emit electromagnetic radiation having a peak emission wavelength between 700-1400 nm;
- a control circuit (120, 130) configured to adjust emission characteristics of the electro-optical element; and
- a driver circuit (110) configured to receive an input from the control circuit (120, 130) and to drive the electro-optical element with a pulsed driving current based on the input,
wherein the apparatus is configured such that the at least one electro-optical element (EL1) emits said electromagnetic radiation only if a visual stimulus at a luminance above 10 cd/m² is present.

2. The apparatus according to claim 1, wherein the apparatus is incorporated in at least one of: a mobile phone, a laptop, a tablet, a monitor, a television, a gaming system, a wearable device, and a computer accessory.

3. The apparatus according to any of the preceding claims, wherein the peak irradiation intensity at an average distance of between 0.1 and 5m from the first electro-optical element (EL1) is 0.1 mW/cm² or more, preferably between 0.4 and 50 mW/cm², and more preferably between 1 and 15 mW/cm².

4. The apparatus according to any of the preceding claims, wherein the radiation emitted by the first electro-optical element (EL1) is directed by optics so that the radiation is emitted from the device having a radiation pattern (220) with a full-width-at-half-power angle of less than 2x60 degrees, preferably less than 2x50 degrees, and more preferably less than 2x25 degrees.

5. The apparatus according to any of the preceding claims, wherein the pulse frequency is 100 Hz or higher.

6. The apparatus according to any of the preceding claims, wherein the pulse frequency is 30 Hz or a multiple of 30 Hz, preferably a common multiple of 24 Hz and 30 Hz, such as 120 Hz.

7. The apparatus according to any of the preceding claims, wherein the control circuit (120, 130) comprises a timer (135) configured to determine an on-period for the electromagnetic radiation emitted by the electro-optical element (EL1), and/or comprises a dose calculation unit configured to determine an accumulated dose of the electromagnetic radiation at the user.

8. The apparatus according to any of the preceding claims, wherein the control circuit (120, 130) is configurable to instruct the driver circuit (110) to shut off the electro-optical element (EL1), if a predetermined maximum on-period is exceeded, and/or if the accumulated dose determined by the dose calculation unit indicates that a predetermined dose of the electromagnetic radiation at the user is exceeded.

9. The apparatus according to claim 8, wherein the control circuit (120, 130) includes a proximity detection unit (130) configured to determine a distance (d) between the user and the apparatus, wherein the control circuit (120, 130) is configured to control the timer and/or the dose calculation unit in dependence on the determined distance (d), such that a delivered dose is between 0.1 and 10 J/cm², which is delivered over a period of 1 hour or longer at an average distance of between 0.2 and 5m from the first light source.

10. The apparatus according to claim 9, wherein the apparatus is configured to provide an irradiated intensity level greater than 1 mW/cm² in a pulsed mode at a duty factor of at least 10% to deliver a dose of more than 1 J/cm² in three hours.

11. A display system comprising:
- the apparatus according to any of claims 1-10; and
- a display screen having a second electro-optical element (EL2) arranged to emit visible light,
wherein the first electro-optical element (EL1) is arranged to emit only if the second electro-optical element (EL2) is emitting with a luminance above 10 cd/m².

12. The display system according to claim 11, wherein the apparatus forms part of the display screen, the display screen comprising:
- a first set of micro-LEDs or VCSELs arranged to emit electromagnetic radiation having a peak emission wavelength between 700 nm and 1400 nm and functioning as said first electro-optical element (EL1), and
- a second set of micro-LEDs to function as display pixels.

13. The display system according to claim 11 or 12, wherein the display screen forms part of a handheld device, such as a mobile phone or a tablet, and wherein the first electro-optical element (EL1) is directed so that the radiation is emitted from the system with a full-width-at-half-power angle of less than 2×60 degrees, preferably less than 2×50 degrees, and more preferably less than 2×25 degrees.

14. The display system according to claim 11 or 12, wherein the display screen forms part of a computer screen, and wherein the radiation emitted by the first electro-optical element is directed so that the radiation is emitted from the system with a full-width-at-half-power angle of less than 2x25 degrees, preferably less than 2x15 degrees.

15. The display system according to claim 11 or 12, wherein the display screen forms part of a television, and wherein the radiation emitted by the first electro-optical element is directed so that the radiation is emitted from the system with a full-width-at-half-power angle of less than 2x15 degrees, preferably less than 2x10 degrees.
